# EUROPEAN PATENT SPECIFICATION

(11) **EP 1 904 517 B1**
(45) Date of publication and mention of the grant of the patent: **21.01.2009**
(21) Application number: 05814210.0
(22) Date of filing: 18.11.2005
(51) Int. Cl.: C07K 7/08, G01N 33/68

(54) **ARTIFICIAL PROTEIN FOR ABSOLUTE QUANTIFICATION OF PROTEINS AND USES THEREOF**
KÜNSTLICHES PROTEIN ZUR ABSOLUTEN QUANTIFIZIERUNG VON PROTEINEN UND VERWENDUNGEN DAVON
PROTEINE ARTIFICIELLE POUR QUANTIFICATION ABSOLUE DE PROTEINES ET UTILISATIONS ASSOCIEES

(30) Priority: 02.06.2005 EP 05090161
(43) Date of publication of application: 02.04.2008
(73) Proprietor: Polyquant GmbH, 93051 Regensburg (DE)
(72) Inventor: PRATT, Julie, M, Wigston, Leicester LE18 1 NP (GB); BEYNON, Robert, Prenton, Merseyside CH43 7PU (GB); GASKELL, Simon, Altrincham, Cheshire WA14 9NJ (GB)
(74) Representative: Elbel, Michaela
(86) International application number: PCT/EP2005/012530
(87) International publication number: WO 2006/128492

(56) References cited:
- WO-A-03/102220
- WO-A-20/04013636
- US-A1- 2002 037 532
- US-A1- 2004 229 283
- ROBERT J BEYNON, MARY K DOHERTY, JULIE M PRATT & SIMON J GASKELL: "Multiplexed absolute quantification in proteomics using artificial QCAT proteins of concatenated signature peptides" NATURE METHODS, vol. 2, no. 8, August 2005 (2005-08), pages 587-589, XP008051422

## Description

### Field of the invention

The present invention relates to proteomics and more specifically to absolute quantification of proteins.

### Background of the invention

The need for absolute quantification in proteomics is becoming increasingly urgent. The most promising method is based on stable isotope dilution involving simultaneous determination of representative proteolytic peptides and stable isotope labeled analogs. The principal limitation to widespread implementation of this approach is the availability of standard signature peptides in accurately known amounts.

The two primary themes in proteomics are protein identification and the comparison of protein expression levels in two physiological or pathological states (comparative proteomics). The long term goal of being able to define the entire proteome of a cell is still unrealized, but the characterization of many thousands of proteins in a single analysis is now attainable.

For proteomics to become a platform technology serving the emergent field of systems biology, there is a pressing need for enhancement of quantification (Righetti, Eur J Mass Spectrom 10 (2004), 335-348). Most comparative proteomics studies deliver *relative* quantification, expressing the changes in amount of a protein in the context of a second cellular state (for example Dunkley, Mol Cell Proteomics (2004); Hoang, J Biomol Tech 14 (2003), 216-233; Ong, Mol Cell Proteomics (2002), 376-386).

However, the goal must ultimately be to define the cellular concentrations of proteins absolutely, whether as molarities or as numbers of molecules per cell. Absolute quantification, which poses one of the greatest challenges in proteomics, draws on well-established precepts in analytical chemistry, and requires either external standards or internal standards (Sechi, Curr Opin Chem Biol 7 (2003), 70-77; Julka, J Proteome Res 3 (2004), 350-363). External standardization is typified by immunodetection, whether solution phase or on position-addressable antibody arrays (Walter, Trends Mol Med 8 (2002), 250-253; Lopez, J Chromatogr B Analyt Technol Biomed Life Sci 787 (2003), 19-27). The second approach, reliant on internal standardization, is based on mass spectrometry (MS), wherein highly selective detection of ions (or ion fragmentations) characteristic of the analytes of interest is combined with the use of internal standards.

In the most rigorous MS analyses, stable isotopic variants of the analytes are used as internal standards. The key underlying principle is that the determination of *relative* signal intensities during mass spectrometric analysis can be converted into *absolute* quantities of analyte by reference to an authentic standard available in known amounts. Direct application of this approach to intact proteins is impractical, and it is common to adopt the principle of surrogacy, that is to quantify indirectly by reference to a proteolytic peptide derived from the protein of interest.

Analyses based on these principles have been dubbed "AQUA" (absolute quantification) using internal standards synthesized *de novo* by chemical methods (Gerber, PNAS 100 (2003), 6940-6945). However, this approach does not lend itself well to absolute quantification of large numbers of proteins, as each Q-peptide would need to be chemically synthesised and independently quantified.

The international patent application PCT/US03/17686, published as WO 03/102220 provides methods to determine the absolute quantity of proteins present in a biological sample. The principle of WO 03/102220 is based on the generation of an ordered array of differentially isotopically tagged pairs of peptides, wherein each pair represents a unique protein, a specific protein isoform or a specifically modified form of a protein. One element of the peptide pairs is a synthetically generated, external standard and the other element of the pair is a peptide generated by enzymatic digestion of the proteins in a sample mixture. For performing the method of WO 03/102220 the standard peptides are calibrated so that absolute amounts are known and added for comparison and quantification. A sample of interest is also labelled with the same isotope tag as used for the standard peptides except differing in the isotopic label. The pairs of signals, which correspond to differentially labelled sample and standard peptides are finally observed and related to a list of expected masses based on the particular standard peptides included. The disadvantage of WO 03/102220 is that standard peptides need to be individually synthesised, purified and quantified. Moreover, both the sample and the standard peptides need to be specifically labelled separately, increasing the potential for variability between experiments.

Thus there is still an existing need to develop easy and convenient methods for absolute quantification in proteomics.

### Summary of the invention

The present invention is directed to an artificial protein for quantitative analysis of the proteome of a sample, cell or organism, comprising the peptides of SEQ ID No:1 to SEQ ID No: 25, wherein the peptides are randowly concatenated

The cleavage sequence between two Q-peptides may be an enzymatic or a chemical cleavage sequence.

The N-terminal and C-terminal extensions protect the quantification peptides from processing and exoproteolysis. The artificial protein further includes features which allow for easy purification of the Q-CAT protein.

Each of the Q-peptides represents one single protein of the sample, cell or organism and each peptide is in a defined stoichiometry, which is typically, but not exclusively 1:1.

It is important to note that the proteins do not have to be purified - partially purified followed by high resolution separation technologies would be equally acceptable.

### Brief description of the drawings

Figure 1 shows examples of Q-peptides selected as signature peptides;
Figure 2 shows the DNA sequence, translated protein sequence and features of the QCAT;
Figure 3 shows the Characterisation of the QCAT protein and Q-peptides;
Figure 4 shows the Quantification using the QCAT protein, and
Figure 5 shows the Use of the QCAT for muscle protein quantification.

### Detailed description of the invention

The inventors describe here the design, expression and use of artificial proteins that are concatamers of tryptic Q-peptides for a series of proteins, generated by gene design *de novo.* The artificial protein comprising the peptides of SEQ ID NO:1 to SEQ ID NO:25, wherein the peptides are randomly concatenated, is designed to include both N-terminal and C-terminal extensions. The function of the extensions is to protect the true Q-peptides, to introduce a purification tag (such as a His-tag) and a sole cysteine residue for quantification of the QCAT.

The novel gene is inserted into a high-level expression vector and expressed in a heterologous expression system such as *E*. *coli.* Within the QCAT protein, each Q-peptide is in a defined stoichiometry (typically, but not exclusively 1:1), such that the entire set of concatenated Q-peptides can be quantified in molar terms by determination of the QCAT protein. Moreover, the QCAT protein is readily produced in unlabelled or labelled form by growth of the expression strain in defined medium containing the chosen label.

The inventors have successfully designed and constructed an artificial gene encoding a concatenation of tryptic peptides (a QCAT protein) from over 20 proteins. The protein further includes features for quantification and purification. The artificial protein was expressed in *E.coli* and synthesis of the correct product was proven by mass spectrometry. The QCAT protein is readily digested with trypsin; is easily quantified and can be used for absolute quantification of proteins. This strategy brings within reach the accurate and absolute quantification of large numbers of proteins in proteomics studies. Additionally, the QCAT was labelled by selective incorporation of a stable isotope labelled amino acid or by incorporation of ¹⁵N nitrogen atoms at every position in the protein.

it is therefore an object of the present invention to provide an artificial protein for quantitative analysis of the proteome of a sample, cell or organism, comprising: the peptides of SEQ ID NO:1 to SEQ ID NO:25, wherein the peptides are randomly concatenated.

In yet another embodiment, the cleavage sequence is cleaved by a protease, preferably by trypsin and the singular marker is a cysteine residue.

Further, one or more peptides of the artificial protein are repeated identically at least one time, preferably one or more times, to achieve a particular stoichiometry between all peptides sequences.

It is preferred to include an affinity tag (e.g. a histidine tag) for purification of the protein. It is particularly preferred to include the affinity tag in either the N-terminal or C-terminal extensions of the protein.

In a further embodiment, the protein is labelled by an isotope, which is selected from the group consisting of ¹³C, ¹⁵N , ²H and ¹⁸O.

In yet a further embodiment each peptide comprises between about 3 and 40 amino acids, preferably about 15 amino acids.

The origin of the proteome, i.e. the sample, cell or organism is preferably a mouse, rat, ape or human, but can be from any proteinaceous source.

In a preferred embodiment of the invention, the peptides represent different conformational, metabolic or modification states of the protein, in order to quantify all proteins derived posttranslationally from such a protein.

Further, the peptides of the artificial protein have preferably a defined molecular weight distribution and quantitative ratios. A mass spectrometer can be calibrated, preferably with molecular weights, which result in equidistant mass spectroscopy signals. Preferably, one or more peptides are represented twice in order to unambiguously label a reference molecular weight for calibration.

Further included in the disclosure of the present invention is the absolute quantification of the protein levels of a certain reference strain, which can then be used to compare the protein levels of this particular strain or similar strains under varying experimental conditions.

The present invention will be better understood by the encompassed examples and results with reference to the accompanying figures.

### Detailed description of the figures

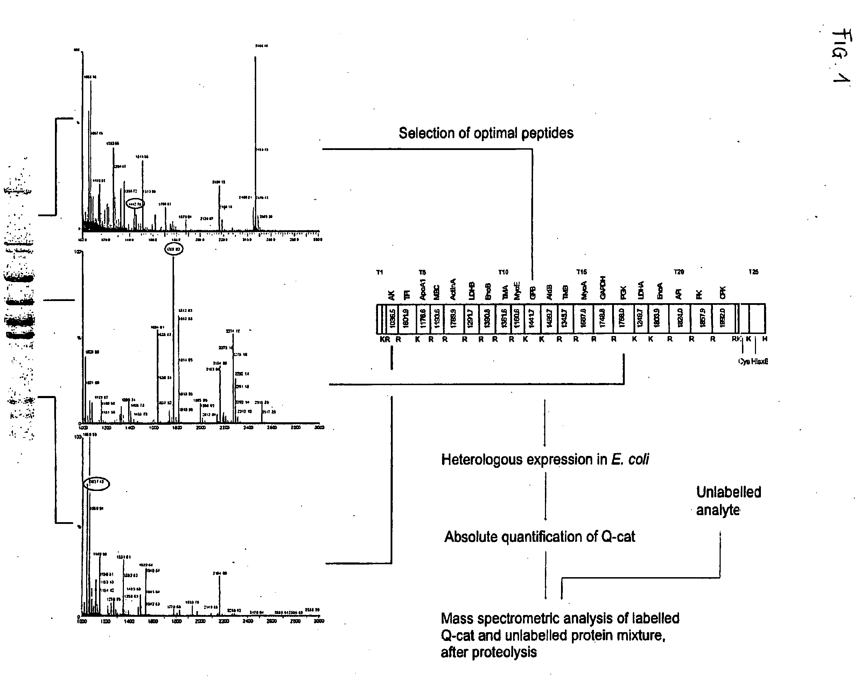

### Figure 1. Examples of Q-peptides selected as signature peptides.

For a series of proteins, peptides were selected (using multiple criteria) from proteins identified as the abundant proteins in a soluble fraction of chicken skeletal muscle, and assembled into an artificial protein, or Q-cat. Left side: Coomassie blue stained, SDS-PAGE analysis of soluble chicken muscle proteins. Center: MALDI-ToF spectrum of tryptic digestion of gel slices corresponding to selected protein bands. The peptide ion labelled with an oval represents the peptide chosen for inclusion in the Q-cat protein, Right side: mass and position of the indicated signature peptides within the designed Q-cat protein. Details of the peptides are in Table 1.

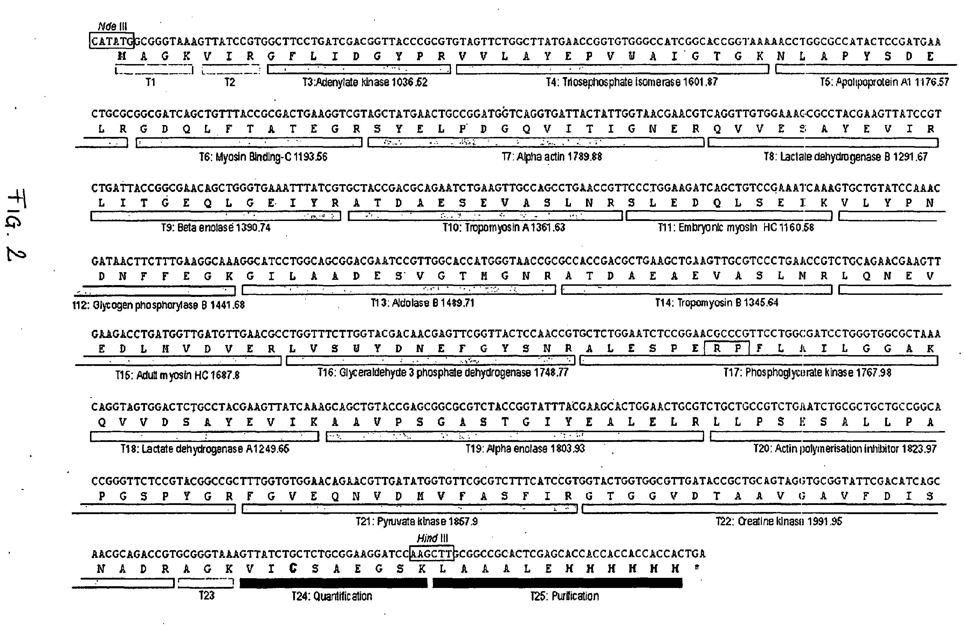

### Figure 2. The DNA sequence, translated protein sequence and features of the QCAT.

The DNA sequence of the synthetic gene, with relevant cloning sites, is shown on the top line and the derived amino acid sequence is shown below. The grey blocked areas indicate the extent of the tryptic peptides, with the donor chicken proteins, tryptic peptide assignment (T1-T25) and the peptide mass (in Da) indicated. A non-cleavable Arg-Pro tryptic site within phosphoglycerate kinase (boxed) is included to confirm the non-digestibility of this site. Peptides (white boxes) encode the initiator methione, N-terminal sacrificial sequence and spacer sequences, and are not derived from proteins of interest. The black boxes highlight the sequences carrying the unique cysteine residue for quantification and His₆ tag for purification. T1 and T2 are sacrificial peptides designed to protect the N-terminus of the first true Q-peptide (T3)

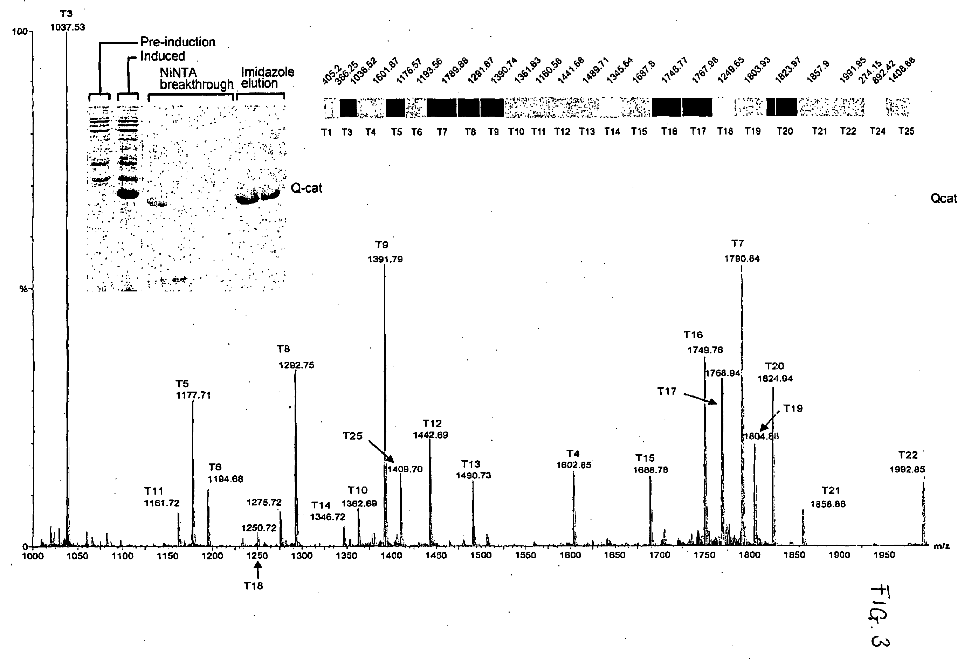

### Figure 3. Characterisation of the QCAT protein and Q-peptides

The pET21a/QCAT plasmid was transformed into *E*. *coli* DE3 cells and after a period of exponential growth, the expression of the QCAT was induced with IPTG. The cell lysates from pre-induced and induced cells were compared on SDS-PAGE (inset). After solubilization of the pellet, and affinity chromatography on a NiNTA column, the purified QCAT protein was homogeneous, and was digested in solution with trypsin. The peptides were analysed on MALDI-ToF mass spectrometry. The inset tryptic digestion map is shaded to indicate the relative intensities of signals corresponding to each peptide in the mass spectrum; peptides smaller than 900 Da, derived from the 'sacrificial' parts of the QCAT are less readily detected in this type of mass spectrometric analysis due to interfering ions.

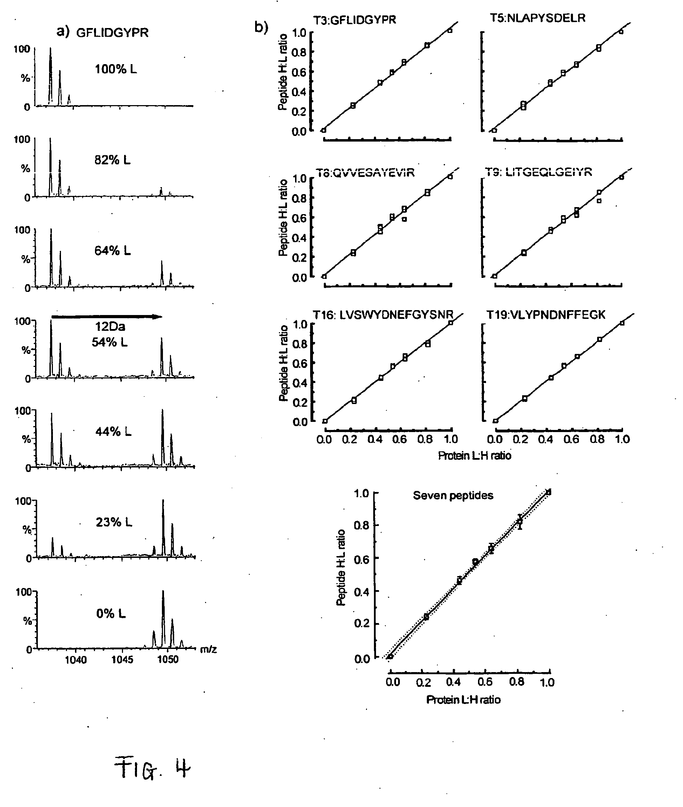

### Figure 4. Quantification using the QCAT protein

The QCAT protein was prepared in unlabelled form (L: "light") and in a form uniformly labelled with ¹⁵N (H: "heavy"). The H and L QCAT proteins were separately purified, quantified and mixed in different ratios, before tryptic digestion and measurement of peptide intensities by MALDI-ToF mass spectrometry. Panel a) illustrates the mass spectrum for the Q-peptide for adenylate kinase (GFLIDGYPR, 12 nitrogen atoms). In panel b) the measured L:H ratios were plotted relative to the mixture ratio, in a triplicate series of experiments for which individual points are shown. In the bottom panel, the data for seven peptides are collated and expressed as mean ± SD (n=18-21). The dotted line defines the 95% confidence limits of the fitted straight line.

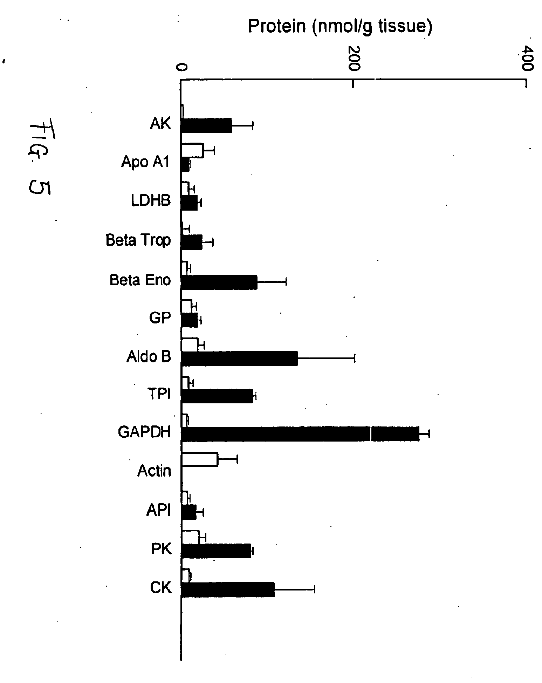

### Figure 5. Use of the QCAT for muscle protein quantification.

A preparation of soluble proteins from skeletal muscle of chicks at 1d and 27d was mixed with [¹⁵N] QCAT, digested with trypsin and analysed by MALDI-ToF MS. For a subset of proteins, it was possible to determine the intensities of the endogenous and standard peptide, and from this, calculate the absolute amounts (in nmol/g tissue) of each protein. Three animals were used at each time point, error bars are SEM (n=3). Proteins were AK: adenylate kinase, ApoA1: apoliporotein A1, LDHB: lactate dehydrogenase B, Beta Trop: beta tropomyosin, Beta Eno: beta enolase, GP: glycogen phosphorylase, ALDO B: aldolase B, TPI: triose phosphate isomerase, GAPDH: glyceraldehyde 3-phosphate dehydrogenase, Actin, API: actin polymerization inhibitor, PK: pyruvate kinase and CK: creatine kinase.

### 1. Design of the gene encoding the Q-protein concatamer

One of the inventors' major interests is in proteome dynamics (Pratt, Mol Cell Proteomics 1 (2002), 579-591), and in changes in protein expression during muscle development (Doherty, Proteomics 4 (2004), 2082-2093; Doherty, Proteomics in press (2005)). A system that shows dramatic developmental changes in protein expression is the chicken pectoralis skeletal muscle from immediately post-hatching to maturity. Accordingly, for the demonstration QCAT set, the inventors chose twenty chicken proteins that had been previously identified as changing in expression level in developing skeletal muscle (Doherty, Proteomics 4 (2004), 2082-2093). A single tryptic fragment was chosen to represent each protein (a "Q-peptide"), although a peptide that can be reproducibly generated by any proteolytic or chemical fragmentation could be used, and in this example, Q-peptide selection was based on theoretical and experimental criteria. The first criterion was that the Q-peptides should lack a cysteine residue, as cysteine residue could be used for quantification of the QCAT and the absence of cysteines should avoid complex intra- and inter-molecular disulphide bond formation in the expressed protein. Secondly, the peptide chosen should be unique within the set of Q-peptides. Thirdly, the Q-peptides were chosen with masses between 1000 Da and 2000 Da, corresponding to the region in MALDI-ToF mass spectra where sensitivity of detection is typically high and interfering signals are low. Finally, an operational criterion was added, inasmuch as the inventors selected peptides that were already demonstrated to give a strong signal on MALDI-ToF mass spectrometry 75% (15 out of 20) of which were Arg-terminated tryptic peptides - the propensity of such peptides to give stronger signals on MALDI-ToF mass spectrometry is well documented (Brancia, Electrophoresis 22 (2001), 552-559). A final, less important criterion was that the Q-peptides should contain at least one instance of an abundant and chemically refractory amino acid such as leucine or valine, as this would facilitate metabolic labeling with amino acids for the preparation of stable isotope labeled Q-peptides. The peptides are summarized in Table 1:

**Table 1 - Peptides selected for Q-cat protein**

| | Peptide mass (Da) | Sequence | N atoms | Parent protein |
|---|---|---|---|---|
| T1 | 405.2 | MAGK | 5 | Construct, sacrificial |
| T2 | 386.25 | VIR | 6 | Construct, sacrificial |
| T3 | 1036.52 | GFLIDGYPR | 12 | Adenylate kinase |
| T4 | 1601.87 | VVLAYEPVWAIGTGK | 16 | Triose phosphate isomerase |
| T5 | 1176.57 | NLAPYSDELR | 14 | Apolipoprotein A1 |
| T6 | 1193.56 | GDQLFTATEGR | 15 | Myosin binding protein C |
| T7 | 1789.88 | SYELPDGQVITIGNER | 21 | Alpha actin |
| T8 | 1291.67 | QVVESAYEVIR | 15 | Lactate dehydrogenase B |
| T9 | 1390.74 | LITGEQLGEIYR | 16 | Beta enolase |
| T10 | 1361.63 | ATDAESEVASLNR | 17 | Alpha tropomyosin |
| T11 | 1160.58 | SLEDQLSEIK | 12 | Myosin heavy chain (embryonic) |
| T12 | 1441.68 | VLYPNDNFFEGK | 15 | Glycogen phosphorylase |
| T13 | 1489.71 | GILAADESVGTMGNR | 19 | Aldolase B |
| T14 | 1345.64 | ATDAEAEVASLNR | 17 | Beta tropomyosin |
| T15 | 1687.8 | LQNEVEDLMVDVER | 19 | Myosin heavy chain (adult) |
| T16 | 1748.77 | LVSWYDNEFGYSNR | 19 | Glyceraldehyde 3-phosphate dehydrogenase |
| T17 | 1767.98 | ALESPERPFLAILGGAK | 21 | Phosphoglycerate kinase |
| T18 | 1249.65 | QVVDSAYEVIK | 13 | Lactate dehydrogenase A |
| T19 | 1803.93 | AAVPSGASTGIYEALELR | 21 | Alpha enolase |
| T20 | 1823.97 | LLPSESALLPAPGSPYGR | 21 | Actin polymerization inhibitor |
| T21 | 1857.9 | FGVEQNVDMVFASFIR | 25 | Pyruvate kinase |
| T22 | 1991.95 | | 25 | Creatine kinase |
| T23 | 274.15 | AGK | 4 | Construct, sacrificial |
| T24 | 892.42 | VICSAEGSK | 10 | Construct, quantification |
| T25 | 1408.68 | LAAALEHHHHHH | 24 | Construct, purification tag |

Once the candidate set was nominated, the Q-peptides were assembled and a gene was constructed, which encoded the assembled Q-peptides using codons for maximal expression in *E*. *coli.* At the C-terminus an extension was added to provide a cysteine residue and a His tag purification motif (the latter provided in this case by the vector pET21a). An additional series of amino acids was appended to the N-terminus to provide an initiator methionine residue and a sacrificial peptide, which when cleaved would expose a true Q-peptide (Figure 1). This avoided complications due to N-formylation or removal of methionine from the N-terminus of QCAT. The transcript encoded by the initial QCAT gene was then analyzed *in silico* for features such as hairpin loops that might compromise translation. If such a feature was noted, the order of the Q-peptides was swapped until an acceptable mRNA structure was obtained - the sequence of Q-peptides within a QCAT is not relevant to their use as quantification standards and the order is thus amenable to such manipulation. The gene was constructed from a series of overlapping oligonucleotides and confirmed by DNA sequencing.

### 2. Expression of the QCAT

The QCAT gene was constructed with restriction sites, such that it could be inserted into a range of expression vectors (Figure 2). In this instance, the gene (confirmed by DNA sequencing) was inserted into pET21a at the *Nde*l and *Hind*lll sites and was expressed initially in *E.coli* (NovaBlue (DE3)) grown in rich medium. After induction by IPTG, SDS-PAGE analysis confirmed high-level expression of a protein of the expected mass (∼35 kDa). This protein was present in the insoluble fraction of sonicated cells, and was presumed to be the QCAT protein present in inclusion bodies. From this preparation we purified the QCAT protein by affinity chromatography using Ni-NTA resin, which resulted in a homogeneous preparation (Figure 2, inset). The intact average mass of this protein, measured by ESI-MS was 33036±2 Da (data not shown), compared to the predicted mass of 33167 Da for the QCAT protein, a difference of 131 Da which is exactly consistent with loss of the methionine residue from the N-terminus. The approx. 35 kDa gel band was subjected to in-gel digestion with trypsin and analysed by MALDI-ToF MS. All predicted QCAT peptides were readily observed in the MALDI-ToF mass spectrum, although the N and C-terminal sacrificial peptidic material yielded, by design, fragments that were too small to be seen in the MALDI-ToF mass spectrum (Figure 3). Although the peptides were chosen to yield good signals on MALDI-ToF, some peptides were markedly less intense than others. These included all of the lysine-terminated peptides, with the exception of T17, which included a non-cleavable Arg-Pro site, which although lysine terminated still yielded a strong signal on MALDI-ToF mass spectrometry. This was particularly evident in the isoform-specific Q-peptides T8 and T18, derived from lactate dehydrogenases A and B (QVVESAYEVIR and QVVDSAYEVIK respectively) - the lysine terminated peptide was less than 10 % of the intensity of the arginine-terminated peptide, which suggests that either Q-peptides should be predominantly drawn from arginine terminated peptides, or alternatively, that a step such as guanidination should be used to convert lysine residues to homoarginine residues, enhancing the propensity to give strong signals (Brancia, Electrophoresis 22 (2001), 552-559). The QCAT was digested by trypsin very effectively and there was no evidence for partial proteolytic products of the Q-peptides, which would of course compromise the quantification step. The inventors then expressed the protein in minimal medium containing ¹⁵NH₄Cl as sole nitrogen source. When digested with trypsin, the resultant MALDI-ToF mass spectrum was of high quality, and all Q-peptides were detectable at the appropriate mass shift corresponding to the number of nitrogen atoms in the peptide (data not shown).

The unlabelled and ¹⁵N-labelled QCAT proteins were then mixed in different ratios, and digested with trypsin before the resultant limit peptides were analysed by MALDI-ToF mass spectrometry. The heavy and light variants of the peptides were readily discerned (Figure 3a) and their intensities measured for a series of peptides (Figure 3b). In all instances, the data were of high quality, and the relationship between proportion of material and the heavy:light ratios was linear, with a slope of one (mean ± SD (n=6) = 1.008 ± 0.008), and very high correlation coefficients (r² greater than 0.99 in all instances). The combined data (Figure 3c) expresses the data for seven peptides; the close boundaries defined by the 95% confidence limits indicate the quality of the quantification.

### Summary of results

The inventors have applied this particular QCAT in the analysis of protein expression in chick skeletal muscle, at 1 d and 27 d post-hatching (Figure 4). Twelve proteins present in this preparation were also represented in the QCAT. MALDI-ToF data of the tryptic peptides was readily acquired, and the changes in protein levels that occur over the first three to four weeks post-hatching were determined.

Because the proteins were absolutely quantified, the inventors were able to express the proteins as nmol per g wet weight of tissue. The variance of the triplicate analyses was small; the inventors attribute this variance to biological rather than analytical variation. The inventors have previously measured the levels of seven of these proteins by 2D gel electrophoresis and densitometry, and the correlation between the quantification using both methods was 0.82 (r², p>0.001). Recognizing that the two methods assess different representations of the proteome, such as charge-variant isoforms or total protein complement, and that the densitometirc method is inevitably imprecise, the correlation is good.

The inventors have demonstrated the feasibility of the QCAT approach for generation of a concatenated set of Q-peptides. The QCAT, designed using both theoretical and experimental considerations, was expressed at high levels, even when grown on minimal medium, and the product was successfully purified. Because the QCAT is a completely artificial construct, the inventors did not anticipate that it would fold into any recognizable three-dimensional structure, and as expected, the protein aggregated into inclusion bodies. This is an advantage as subsequent purification is simpler, only requiring resolubilization of the pellet in strong chaotropes prior to affinity purification. Further, the lack of higher order structure of the QCAT would ensure that the QCAT was digested at least as quickly as the target proteins to be quantified.

### Use of the artificial protein and the QCAT peptides

There are two ways in which the concatamers will be used. First, in the direct Q-peptide method, the concatamers are used as an internal standard. The stable-isotope labelled concatamer can be directly added to a sample or cell preparation before the proteolysis step. Alternatively, for some cell systems, the concatamer quantification can be used to achieve absolute quantification of a reference strain grown under carefully defined conditions - the **indirect Q-peptide** approach. This reference strain can then be used, in stable isotope labelled form, as an absolute quantification standard for all future proteomics quantification studies using that organism. Once a reference strain is accurately quantified, any peptide can be used to report on a protein, rather than the restricted set used as Q-peptides and this is clearly a very attractive proposition. This extends the generality of different proteomic strategies, and creates a new niche for tagging methods such as ICAT (Gygi, J Proteome Res 1 (2002), 47-54) and ITRAQ (Ross, Molecular and Cellular Proteomics in press (2004)) in a comparative proteomics analysis of an unknown against a fully quantified strain.

However, the inventors recognize that there are many instances where this approach is not appropriate, and where a stable isotope labelled concatamer itself (the **direct Q-peptide** approach) will be the appropriate standard. This is particularly apposite in proteomics studies using biological material that cannot be readily pre-labelled, for example, in animal tissues or in biomarker studies.

### Particular advantages

The strategy the inventors advocate is superior to chemical synthesis of each individual Q-peptide, usually in a stable isotope form. Whilst chemical synthesis has been used in one-off applications, the process of peptide synthesis is not sufficiently 'clean' as to obviate exhaustive purification of the product. Secondly, for multiplexed assays, each peptide would need to be individually quantified before use. Finally, chemically synthesized Q-peptides are a finite resource whereas repeated expression of the QCAT gene is facile. High quality absolute quantification may be an effective route to overcome the difficulties associated with current methods for comparative proteomics, whether based on gel analysis or mass spectrometry. A series of comparative studies of particular cellular systems, each by comparison to a QCAT quantified reference would not only be individually quantified, but should be sufficiently rigorous that as the data sets grow, any pair-wise comparison would be robust, transferable between individual laboratories and stable over time.

It should be possible to factor in the propensity of the peptide to ionize and generate a good signal in the mass spectrometer. At present, ion intensities are not used exhaustively in the analysis of mass spectra, although there have been some recent attempts to predict intensity using knowledge-based approaches (Krause, Anal Chem 71 (1999), 4160-4165; Gay, Proteomics 2 (2002), 1374-1391; Baumgart, Rapid Commun Mass Spectrom 18 (2004), 863-868).

An additional factor that must be taken into account is the choice of precursor label. Whilst uniform labeling with [¹³C] or [¹⁵N] ensures that every peptide is comprehensively labeled, it might be preferable to select Q-peptides that each contain the same amino acid that is then used as the stable isotope labeled precursor. Since most QCAT proteins would be anticipated to be assemblies of tryptic peptides, a strategy of incorporation of [¹³C₆]-lysine and [¹³C₆]-arginine would also ensure that most Q-peptides would be singly labeled and the mass offset between heavy and light peptides would be a constant 6 Da. Further, an unlabelled QCAT could be labeled *in vitro* using reagents advocated for comparative proteomics, enhancing all of these technologies to absolute quantification.

Without being bound by any particular theory, the inventors believe that the number of Q-peptides that could be assembled into a single QCAT is limited by the ability to achieve high-level heterologous expression of large proteins. In the example given here, the inventors chose 20 peptides of average length 15 amino acids and average molecular weight 1.5 kDa. If 100 proteins were represented in a single QCAT, the resultant recombinant protein would be 150 kDa, which should be readily expressed. The entire yeast proteome, of approx 6000 proteins, could then be defined within approx. 60 QCAT constructs.

This ability to quantify as many as 100 proteins in a single construct invites the challenge of optimal assembly of individual Q-peptides in QCATs. Different criteria might be envisaged. First, a group of Q-peptides would allow absolute quantification of a particular subcellular fraction, or of a specific subset of proteins, for example transcription factors or protein kinases. Secondly, and perhaps more importantly, concatenation could be driven by the abundance of the target proteins in the cell. It would be difficult to quantify two different proteins with widely different expression levels in the same QCAT experiment, and it might be preferable to assemble high abundance proteins in a construct distinct from that encoding low abundance proteins.

Other applications for QCATs are readily envisaged, particularly in the broad areas of clinical biology and toxicology and diagnostics. Absolute quantification will add a new dimension to the predictive values of analyses in clinical or other biomarker monitoring systems and will absolutely define the stoichiometric ratios of individual proteins within a subcellular compartment or a multi-protein complex.

### Materials and methods

**Materials**. [¹⁵N]H₄Cl (99% atom percent excess) was provided by CK Gas Products Ltd. Hampshire, UK. Most reagents, except where listed here, have been described previously (Doherty, Proteomics 4 (2004) 2082-2093; Pratt, Proteomics 2 (2002), 157-160). Chick (layer, Hi-Sex Brown) skeletal muscle proteins were prepared from 1 d and 27 d old chicks as a 20.000g supernatant of a 10 % (w/v) homogenate (Doherty, Proteomics 4 (2004) 2082-2093; Doherty, Proteomics 5 (2005) 5, 522-533.

**QCAT gene design and construction**. Q-peptides were selected for uniqueness of mass, propensity to ionise and be detectable in mass spectrometry, the presence of specific amino acid residues (for example, leucine or valine), the absence of other amino acid residues (cysteine, histidine, methionine). The peptide sequences were then randomly concatenated *in silico* and used to direct the design of a gene, codon-optimised for expression in *E*. *coli.* The predicted transcript was analysed for RNA secondary structure that might diminish expression, and if this was present, the order of the peptides was altered. N-and C-terminal sequences were added as sacrificial structures, protecting the assembly of true Q-peptides from exoproteolytic attack during expression. Additional peptide sequences were added to provide an initiator methionine and a C-terminal cysteine residue for quantification. The artificial gene was synthesised *de novo* (by Entelechon GmbH, Germany) from a series of overlapping oligonucleotides, verified by DNA sequencing and ligated into the *Nde*I and *Hind*III sites of the pET21a expression vector, to yield the QCAT plasmid, pET21a QCAT. A His₆ purification tag was provided by fusion to the vector.

**QCAT gene expression and labeling with ¹⁵N.** The QCAT plasmid, pET21a QCAT, was used to transform NovaBlue (DE3) (K-12 *endA1, hsdR17*(*r_{K12}⁻mK₁₂*⁺), *supE44, thi-1, recA1, gyrA96, relA1, lac,* F'[*proA*⁺*B*⁺, *Iacl^{q}Z*□*M15*::Tn *10*(Tc^{R})] cells to ampicillin resistance. Cells were grown at 37°C in Luria broth, 100 µg/ml ampicillin to an A₆₀₀ of 0.4-0.6 and IPTG added to 1 mM. Incubation continued for a further five hours when cells were pelleted by centrifugation (5.000g, 4 min., 4°C), resuspended in 10 ml 20 mMTris/HCl buffer, pH 8.0 and lysozyme was added (100 µg/ml) for ten minutes at room temperature. Cells were then sonicated (three bursts of 30s) on ice and centrifuged at 14000 g for 10min. Pellets and supernatants of induced and uninduced cultures were analysed by 12,5 % (w/v) SDS PAGE/Coomassie blue staining. For ¹⁵N-labelling, cells were grown in M9 minimal medium prepared using [¹⁵N]H₄Cl (20 mM), and induced and processed as above.

Purification **and analysis of the QCAT protein.** The pellets from sonicated cells were dissolved in 20 mM phosphate buffer (pH 7.5) containing 20 mM imidazole and 8 M urea (Buffer A) before being applied to a NiNTA column (GE Healthcare). After 10 column volumes of washing in the same buffer, the bound material was eluted with buffer A with an increased concentration of imidazole (500 mM). This material was desalted on Sephadex G25 'spun columns' and the mass of the eluted protein was determined by electrospray ionisation mass spectrometry using a Waters-Micromass Q-ToF micro mass spectrometer. The mass spectra were processed using the MaxEnt I algorithm. The purified desalted protein was digested with trypsin, and the resultant peptides were mass measured using a Waters-Micromass MALDI-ToF mass spectrometer (Doherty, Proteomics 4 (2004) 2082-2093). To assess the response ratio of heavy and light variants of the QCAT, the purified 'heavy' and 'light' proteins were mixed in different ratios prior to digestion with trypsin and MALDI-ToF mass spectrometry. The intensities of the [¹⁴N]- and [¹⁵N]- peptides were measured on centroided spectra.

**Use of the QCAT to quantify muscle protein expression.** The supernatant fraction containing chicken soluble proteins derived from 100mg of tissue was mixed with 290 µg of [¹⁵N] QCAT, quantified by protein assay and digested with trypsin overnight - the QCAT was digested at a higher rate than endogenous muscle proteins (results not shown). The experiment was replicated for three animals at each time point. Subsequently, the [¹⁴N]- (muscle) and [¹⁵N]- (QCAT) peptides were identified by mass, and their relative intensities measured by MALDI-ToF mass spectrometry.

### Mass Spectrometric Analysis

The analysis in this example was carried out using a MALDI-ToF Mass Spectrometer, but this method is equally applicable to all other Mass Spectrometric methods suitable for the analysis of peptides.

### References

Baumgart, S. et al. The contributions of specific amino acid side chains to signal intensities of peptides in matrix-assisted laser desorption/ionization mass spectrometry. Rapid Commun Mass Spectrom 18, 863-868 (2004).
Brancia, F.L. et al. A combination of chemical derivatisation and improved bioinformatic tools optimises protein identification for proteomics. Electrophoresis 22, 552-559 (2001).
Doherty, M.K. et al. The proteome of chicken skeletal muscle: changes in soluble protein expression during growth in a layer strain. Proteomics 4, 2082-2093 (2004).
Doherty, M.K., Whitehead, C., McCormack, H., Gaskell, S.J. & Beynon, R.J. Proteome dynamics in complex organisms: using stable isotopes to monitor individual protein turnover rates. Proteomics 5, 522-533. (2005)
Dunkley, T.P., Watson, R., Griffin, J.L., Dupree, P. & Lilley, K.S. Localization of organelle proteins by isotope tagging (LOPIT). Mol Cell Proteomics (2004). Gay, S., Binz, P.A., Hochstrasser, D.F. & Appel, R.D. Peptide mass fingerprinting peak intensity prediction: extracting knowledge from spectra. Proteomics 2, 1374-1391 (2002).
Gerber, S.A., Rush, J., Stemman, O., Kirschner, M.W. & Gygi, S.P. Absolute quantification of proteins and phosphoproteins from cell lysates by tandem MS. Proc Natl Acad Sci U S A 100, 6940-6945 (2003).
Gygi, S.P., Rist, B., Griffin, T.J., Eng, J. & Aebersold, R. Proteome analysis of low-abundance proteins using multidimensional chromatography and isotope-coded affinity tags. J Proteome Res 1, 47-54 (2002).
Hoang, V.M. et al. Quantitative proteomics employing primary amine affinity tags. J Biomol Tech 14, 216-223 (2003).
Julka, S. & Regnier, F. Quantification in proteomics through stable isotope coding: a review. J Proteome Res 3, 350-363 (2004).
Krause, E., Wenschuh, H. & Jungblut, P.R. The dominance of arginine-containing peptides in MALDI-derived tryptic mass fingerprints of proteins. Anal Chem 71, 4160-4165 (1999).
Lopez, M.F. & Pluskal, M.G. Protein micro- and macroarrays: digitizing the proteome. J Chromatogr B Analyt Technol. Biomed Life Sci 787, 19-27 (2003). Ong, S.E. et al. Stable isotope labeling by amino acids in cell culture, SILAC, as a simple and accurate approach to expression proteomics. Mol Cell Proteomics 1, 376-386 (2002).
Pratt, J.M. et al. Stable isotope labelling in vivo as an aid to protein identification in peptide mass fingerprinting. Proteomics 2, 157-163 (2002).
Pratt JM, Petty J, Riba-Garcia I, Robertson DH, Gaskell SJ, Oliver SG, Beynon RJ. Dynamics of protein turnover, a missing dimension in proteomics.Mol Cell Proteomics. 2002 Aug;1 (8):579-991 (2002).
Righetti, P.G., Campostrini, N., Pascali, J., Hamdan, M. & Astner, H. Quantitative proteomics: a review of different methodologies. Eur J Mass Spectrom (Chichester, Eng) 10, 335-348 (2004).
Ross, P.L. et al. Multiplexed protein quantitiation in Saccharomyces cerevisiae using amine-reactive isobaric tagging reagents. Molecular and Cellular Proteomics (in press) (2004).
Sechi, S. & Oda, Y. Quantitative proteomics using mass spectrometry. Curr Opin Chem Biol 7, 70-77 (2003).
Walter, G., Bussow, K., Lueking, A. & Glokler, J. High-throughput protein arrays: prospects for molecular diagnostics. Trends Mol Med 8, 250-253 (2002).
WO 03/102220 (Aebersold, R.)

## Claims

1. Artificial protein, comprising the peptides of SEQ ID NO:1 to SEQ ID NO: 25, wherein the peptides are randomly concatenated.

2. Artificial protein of claim 1, for quantitative analysis of the proteome of a sample, cell or organism comprising the sequence of Figure 2.

3. Artificial protein of claim 1, wherein the order of the peptides is altered if an RNA secondary structure is present which might diminish expression.

4. Nucleotide sequence coding for an artificial protein comprising the sequence of Figure 2.

5. Use of an artificial protein of any of claims 1 to 3 as an internal standard.

6. Use of an artificial protein of any of claims 1 to 3 as an absolute quantification standard.

## Patentansprüche

1. Künstliches Protein, das die Peptide von SEQ ID NR: 1 bis SEQ ID NR: 25 umfasst, wobei die Peptide zufällig konkatamerisiert sind.

2. Künstliches Protein nach Anspruch 1 zur quantitativen Analyse des Proteoms einer Probe, einer Zelle oder eines Organismus, das die Sequenz von Figur 2 umfasst.

3. Künstliches Protein nach Anspruch 1, wobei die Reihenfolge der Peptide verändert ist, wenn eine sekundäre RNA Struktur vorhanden ist, die eine Expression abschwächen könnte.

4. Nukleotidsequenz, die ein künstliches Protein kodiert, das die Sequenz von Figur 2 umfasst.

5. Verwendung eines künstlichen Proteins nach einem der Ansprüche 1 bis 3 als ein interner Standard.

6. Verwendung eines künstlichen Proteins nach einem der Ansprüche 1 bis 3 als ein absoluter Quantifizierungsstandard.

## Revendications

1. Protéine artificielle comprenant les peptides SEQ ID NO:1 à SEQ ID NO :25, dans laquelle les peptides sont concaténés de façon aléatoire.

2. Protéine artificielle selon la revendication 1, pour l'analyse quantitative du protéome d'un échantillon, d'une cellule ou d'un organisme comprenant la séquence de la Figure 2.

3. Protéine artificielle selon la revendication 1, dans laquelle l'ordre des peptides est altéré si une structure secondaire d'ARN pouvant diminuer l'expression est présente.

4. Séquence nucléotidique codant pour une protéine artificielle comprenant la séquence de la Figure 2.

5. Utilisation d'une protéine artificielle selon l'une quelconque des revendications 1 à 3 en tant que standard interne.

6. Utilisation d'une protéine artificielle selon l'une quelconque des revendications 1 à 3 en tant que standard de quantification absolu.


### SEQUENCE LISTING

<110> Entelechon GmbH
<120> Artificial protein, method for absolute quantification of proteins and uses thereof
<130> E30006
<160> 25
<170> PatentIn version 3.1
<210> 1
   <211> 4
   <212> PRT
   <213> Artificial - Sacrificial construct I
<400> 1
   Met Ala Gly Lys
   1
<210> 2
   <211> 3
   <212> PRT
   <213> Artificial - Sacrificial construct II
<400> 2
   Val Ile Arg
   1
<210> 3
   <211> 9
   <212> PRT
   <213> Artificial - Adenylate kinase
<400> 3
   Gly Phe Leu Ile Asp Gly Tyr Pro Arg
   1               5
<210> 4
   <211> 15
   <212> PRT
   <213> Artificial - Triose phosphate isomerase
<400> 4
   Val Val Leu Ala Tyr Glu Pro Val Trp Ala Ile Gly Thr Gly Lys
   1               5                   10                  15
<210> 5
   <211> 10
   <212> PRT
   <213> Artificial - Apolipoprotein A1
<400> 5
   Asn Leu Ala Pro Tyr Ser Asp Glu Leu Arg
   1               5                   10
<210> 6
   <211> 11
   <212> PRT
   <213> Artificial - Myosin binding protein C
<400> 6
   Gly Asp Gln Leu Phe Thr Ala Thr Glu Gly Arg
   1               5                   10
<210> 7
   <211> 16
   <212> PRT
   <213> Artificial - Alpha actin
<400> 7
   Ser Tyr Glu Leu Pro Asp Gly Gln Val Ile Thr Ile Gly Asn Glu Arg
   1               5                   10                  15
<210> 8
   <211> 11
   <212> PRT
   <213> Artificial - Lactate dehydrogenase B
<400> 8
   Gln Val Val Glu Ser Ala Tyr Glu Val Ile Arg
   1               5                   10
<210> 9
   <211> 12
   <212> PRT
   <213> Artificial - Beta enolase
<400> 9
   Leu Ile Thr Gly Glu Gln Leu Gly Glu Ile Tyr Arg
   1               5                   10
<210> 10
   <211> 13
   <212> PRT
   <213> Artificial - Alpha tropomyosin
<400> 10
   Ala Thr Asp Ala Glu Ser Glu Val Ala Ser Leu Asn Arg
   1 5 10
<210> 11
   <211> 10
   <212> PRT
   <213> Artificial - Myosin heavy chain (em-bryonic)
<400> 11
   Ser Leu Glu Asp Gln Leu Ser Glu Ile Lys
   1 5 10
<210> 12
   <211> 12
   <212> PRT
   <213> Artificial - Glycogen phosphorylase
<400> 12
   Val Leu Tyr Pro Asn Asp Asn Phe Phe Glu Gly Lys
   1 5 10
<210> 13
   <211> 15
   <212> PRT
   <213> Artificial - Aldolase B
<400> 13
   Gly Ile Leu Ala Ala Asp Glu Ser Val Gly Thr Met Gly Asn Arg
   1 5 10 15
<210> 14
   <211> 13
   <212> PRT
   <213> Artificial - Beta tropomyosin
<400> 14
   Ala Thr Asp Ala Glu Ala Glu Val Ala Ser Leu Asn Arg
   1 5 10
<210> 15
   <211> 14
   <212> PRT
   <213> Artificial - Myosin heavy chain (adult)
<400> 15
   Leu Gln Asn Glu Val Glu Asp Leu Met Val Asp Val Glu Arg
   1 5 10
<210> 16
   <211> 14
   <212> PRT
   <213> Artificial - Glyceraldehyde 3-phosphate dehydro-genase
<400> 16
   Leu Val Ser Trp Tyr Asp Asn Glu Phe Gly Tyr Ser Asn Arg
   1 5 10
<210> 17
   <211> 17
   <212> PRT
   <213> Artificial - Phosphoglycerate kinase
<400> 17
   Ala Leu Glu Ser Pro Glu Arg Pro Phe Leu Ala Ile Leu Gly Gly Ala
   1 5 10 15
   Lys
<210> 18
   <211> 11
   <212> PRT
   <213> Artificial - Lactate dehydrogenase A
<400> 18
   Gln Val Val Asp Ser Ala Tyr Glu Val Ile Lys
   1 5 10
<210> 19
   <211> 18
   <212> PRT
   <213> Artificial - Alpha enolase
<400> 19 Leu Arg
<210> 20
   <211> 18
   <212> PRT
   <213> artificial - Actin polymerization inhibitor
<400> 20
   Leu Leu Pro Ser Glu Ser Ala Leu Leu Pro Ala Pro Gly Ser Pro Tyr
   1 5 10 15
   Gly Arg
<210> 21
   <211> 16
   <212> PRT
   <213> Artificial - Pyruvate kinase
<400> 21
   Phe Gly Val Glu Gln Asn Val Asp Met Val Phe Ala Ser Phe Ile Arg
   1 5 10 15
<210> 22
   <211> 21
   <212> PRT
   <213> Artificial - Creatine kinase
<400> 22
   Gly Thr Gly Gly Val Asp Thr Ala Ala Val Gly Ala Val Phe Asp Ile
   1 5 10 15
   Ser Asn Ala Asp Arg
   20
<210> 23
   <211> 3
   <212> PRT
   <213> Artificial - Sacrificial construct III
<400> 23
   Ala Gly Lys
   1
<210> 24
   <211> 9
   <212> PRT
   <213> Artificial - Quantification construct
<400> 24
   Val Ile Cys Ser Ala Glu Gly Ser Lys
   1               5
<210> 25
   <211> 12
   <212> PRT
   <213> Artificial - Purification tag construct
<400> 25
   Leu Ala Ala Ala Leu Glu His His His His His His
   1               5                   10